# EUROPEAN PATENT APPLICATION

(11) **EP 1 927 654 A1**
(43) Date of publication of application: **04.06.2008**
(21) Application number: 06291855.2
(22) Date of filing: 01.12.2006
(51) Int. Cl.: C12N 1/18, C12N 1/19, C12P 11/00, C12G 1/00

(54) **Use of ure2 mutant yeasts for increasing the release of aromatic volatile thiols by yeast during fermentation**

(71) Applicant: Sarco, 33015 Bordeaux Cedex (FR)
(72) Inventor: Marullo, Philippe, 33370 Tresses (FR); Thibon, Cécile, 33800 Bordeaux (FR); Dubourdieu, Denis, 33410 Beguey (FR); Tominaga, Takatoshi, 33000 Bordeaux (FR)
(74) Representative: Leblois-Préhaud, Hélène Marthe Georgette

(57) **Abstract**

Use of a *URE2* mutant yeast having a loss-of-function mutation in the *URE2* gene for increasing the release of aromatic volatile thiol by yeast during fermentation.

SEQUENCE LISTING

<110>  SARCO

<120>  Use of URE2 mutant yeasts for increasing the release of aromatic volatile
       thiols by yeast during fermentation

<130>  s2012EP1

<160>  5

<170>  PatentIn version 3.3

<210>  1
<211>  354
<212>  PRT
<213>  Saccharomyces cerevisiae

<400>  1

Met Met Asn Asn Asn Gly Asn Gln Val Ser Asn Leu Ser Asn Ala Leu
1               5                   10                  15

Arg Gln Val Asn Ile Gly Asn Arg Asn Ser Asn Thr Thr Thr Asp Gln
            20                  25                  30

Ser Asn Ile Asn Phe Glu Phe Ser Thr Gly Val Asn Asn Asn Asn Asn
        35                  40                  45

Asn Asn Ser Ser Ser Asn Asn Asn Asn Val Gln Asn Asn Asn Ser Gly
    50                  55                  60

Arg Asn Gly Ser Gln Asn Asn Asp Asn Glu Asn Asn Ile Lys Asn Thr
65                  70                  75                  80

Leu Glu Gln His Arg Gln Gln Gln Gln Ala Phe Ser Asp Met Ser His
                85                  90                  95

Val Glu Tyr Ser Arg Ile Thr Lys Phe Phe Gln Glu Gln Pro Leu Glu
            100                 105                 110

Gly Tyr Thr Leu Phe Ser His Arg Ser Ala Pro Asn Gly Phe Lys Val
        115                 120                 125

Ala Ile Val Leu Ser Glu Leu Gly Phe His Tyr Asn Thr Ile Phe Leu
    130                 135                 140

Asp Phe Asn Leu Gly Glu His Arg Ala Pro Glu Phe Val Ser Val Asn
145                 150                 155                 160

Pro Asn Ala Arg Val Pro Ala Leu Ile Asp His Gly Met Asp Asn Leu
                165                 170                 175

Ser Ile Trp Glu Ser Gly Ala Ile Leu Leu His Leu Val Asn Lys Tyr
            180                 185                 190

Tyr Lys Glu Thr Gly Asn Pro Leu Leu Trp Ser Asp Asp Leu Ala Asp
        195                 200                 205

Gln Ser Gln Ile Asn Ala Trp Leu Phe Phe Gln Thr Ser Gly His Ala
    210                 215                 220

Pro Met Ile Gly Gln Ala Leu His Phe Arg Tyr Phe His Ser Gln Lys
225                 230                 235                 240

Ile Ala Ser Ala Val Glu Arg Tyr Thr Asp Glu Val Arg Arg Val Tyr
                245                 250                 255

Gly Val Val Glu Met Ala Leu Ala Glu Arg Arg Glu Ala Leu Val Met
            260                 265                 270

Glu Leu Asp Thr Glu Asn Ala Ala Ala Tyr Ser Ala Gly Thr Thr Pro
        275                 280                 285

Met Ser Gln Ser Arg Phe Phe Asp Tyr Pro Val Trp Leu Val Gly Asp
    290                 295                 300

Lys Leu Thr Ile Ala Asp Leu Ala Phe Val Pro Trp Asn Asn Val Val
305                 310                 315                 320

Asp Arg Ile Gly Ile Asn Ile Lys Ile Glu Phe Pro Glu Val Tyr Lys
                325                 330                 335

Trp Thr Lys His Met Met Arg Arg Pro Ala Val Ile Lys Ala Leu Arg
            340                 345                 350

Gly Glu

<210>  2
<211>  22
<212>  DNA
<213>  artificial sequence

<220>
<223>  primer

<400>  2
caaatgccga gaaaaatacc gc                                              22

<210>  3
<211>  21
<212>  DNA
<213>  artificial sequence

<220>
<223>  primer

<400>  3
aaacgaacgc cgaaacacat a                                               21

<210>  4
<211>  21
<212>  DNA
<213>  artificial sequence

<220>
<223>  primer

<400>  4
gagcagattg ttttgagtag g                                                21


<210>  5
<211>  20
<212>  DNA
<213>  artificial sequence

<220>
<223>  primer

<400>  5
ttcctaagca actcatcgtg                                                  20

## Description

The invention relates to the use of *URE2* mutant yeasts for increasing the release of aromatic volatile thiols by yeast during fermentation and its application for improving the aroma of fermented products, in particular alcoholic beverages produced by yeast fermentation (wine, cider, beer), as well as for developing yeast starter strains with optimized volatile thiol release capabilities.

Volatile thiols are extremely aromatic molecules that can affect the aroma of numerous foods such as fruits and grilled meat (Shankaranarayana et al.. Sulphur compounds in flavours. In Food Flavors; Morton, I.D., Macleod, A.J., Eds; Elsevier Science Publishers: Amsterdam, 1982; part A, pp169-281). Several compounds in this family, play a decisive role in contributing to the aroma of wines made from the Sauvignon Blanc grape variety (Tominaga et al., J. Agric. Food Chem., 1998c, 46: 5215-5219). Their aromatic contribution was also successively demonstrated in several other grape varieties such as Gewürztraminer, botrytized Sémillon, Colombard, Petit Manseng (Tominaga et al., Am. J. Enol. Vitic., 2000a, 51, 178-181), and Merlot (Aznar et al., J. Agric. Food Chem., 2000, 49, 2924-2929). Among these compounds, 4-mercapto-4-methyl-pentan-2-one (4MMP) (Darriet et al., Flavour Fragr. J., 1995, 10, 385-392; *Tominaga et al*., 1998c, precited) and 3-mercaptohexan-1-ol (3MH) (Tominaga et al., Flavour Fragr. J., 1998a, 13, 159-162) are principally formed during alcoholic fermentation from inodorous non-volatile specific *S*-cysteine conjugate precursors present in the must (Tominaga et al., J. Int. Sci. Vigne Vin., 1995, 29, 227-232; *Tominaga et al.,* 1998a). The transformation of such precursors in volatile thiols is achieved by yeast metabolism and involves a carbon-sulfur β-lyase activity (Tominaga *et al.,* 1998c ; Wakabayashi et al., J. Agric. Food Chem., 2004, 52, 110-116). The 3-mercaptohexyl acetate (3MHA or A3MH) resulting from the acetylation of 3MH by yeast also contributes to wine aroma.

4MMP, 3MHA, and 3MH are characterized by fruity descriptors (box tree, passion fruit, and grapefruit, respectively) and present very low perception thresholds (0.8, 4.2, and 60 ng L⁻¹, respectively; *Tominaga et al.,* 1998a). In addition, 3MH and 3MHA are chiral molecules with 2 enantiomers, *R* and *S*. In dry white wine made from healthy grapes, 3MH is almost racemic (*R* and *S* ratio around 50:50), whereas 3MHA is mainly in the *S* form (*R* and *S* ratio around 30:70). In wines made from botyrized grapes (*Botrytis cinerea*), the enantiomer distribution of 3MH is 30:70 in favor of the S form. It was recently demonstrated that 3MH(*R*) and 3MH(*S*) enantiomers produced different aromatic nuances (grape fruit and passion fruit, respectively) with similar perception thresholds in hydroalcoholic model solution (50 and 60 ng L, respectively). The perception thresholds of the *R* and *S* enantiomers of 3MHA are slightly different (9 and 2.5 ng/L): the less odoriferous R form is reminiscent of passion fruit, while the *S* form has a more herbaceous odor of boxwood (Tominaga et al., J. Agric. Food. Chem., 2006, 54, 7251-7255).

The powerful aromatic properties of these molecules prompted recent studies to understand how their production can be enhanced and modulated in winemaking. Viticultural practices such as nitrogen feeds (Choné et al., J. Int. Sci. Vigne Vin., 2006, 40, 1-6) and pre-fermentation operations such as skin contact (Peyrot des Gachons et al., Am. J. Enol., Vitic., 2002, 53, 144-156) modulate the amount of aromatic precursors in grape must. Fermentation conditions such as temperature (Howell et al., FEMS Microbiol. Letter, 2004, 240, 125-129; Masneuf-Pomarède et al., Int J. Food Microbiol., 2006, 108, 385-390) also influence the final concentration of these aromas in wine. However, the most important factor in thiol liberation is undoubtedly the yeast strain (Dubourdieu et al., Am. J. Enol. Vitic., 2006, 57, 81-88). A strong variability (about tenfold) was found among industrial wine starter strains of *S*. *cerevisiae* (Murat et al., 2001, Am. J. Enol., 52, 136-140; Howell *et al.,* 2004, precited; Masneuf-Pomarède et al., Int. J. Food Microbiol., 2006, 108, 385-390), *S. uvarum,* and related inter-specific hybrids (Masneuf et al., J. Int. Sci. Vigne Vin, 2002, 36, 205-212). This variability induced strain screening and breeding programs to obtain new strains having higher thiol liberation (Murat *et al*., 2001; Masneuf *et al*., 2002, precited). However, the genetic determinism and molecular mechanisms of thiol liberation by yeast remains unclear.

Recently, Howell *et al.* identified three genes *BNA3, CYS3,* and *IRC7* that encode putative β-lyase in the *Saccharomyces cerevisiae* genome. Using gene deletion experiments in a wine yeast strain background, they suggested that these enzymes are able to cleave 4MMP-precursor into the related volatile aroma during fermentation in synthetic grape juice (Howell et al., Applied Environ. Microbiology, 2005, 71, 5420-5426). Among these genes, *BNA3* encoding for an arylformamidase was found to contribute strongly to volatile thiol release. Although these enzymes have been identified, the mechanisms of precursor assimilation and cleavage by yeast during fermentation are far from being well-understood.

Ure2p is a transcriptional regulator of genes involved in nitrogen metabolism in yeast, acting as negative regulator of Nitrogen Catabolite Repression (NCR)-sensitive genes (Cooper, T. G. and Sumrada, R.A., J. Bacteriol., 1983, 155, 623-627; Coschinago, P.W. and Magasanik, B., Mol. Cell. Biol., 1991, 11, 822-832; Ter Schure et al., FEMS Microbiol. Reviews, 2000, 24, 67-83; Magasanik, B., P.N.A.S., 2005, 102, 16537-16538; Cooper T., 1982, Transport in Saccharomyces cerevisiae. In The molecular biology of yeast Saccharomyces metabolism and gene expression. Strathern, J.N., Laboratory CSH, Eds, pp. 399-461). Nitrogen Catabolite Repression (NCR) is the mechanism by which a yeast is able to select nitrogen sources enabling the best growth (preferred nitrogen source, good nitrogen source or rich nitrogen source : glutamate, glutamine, ammonia and asparagine) over non-preferred nitrogen sources (poorer nitrogen source : urea, gamma-amino-butyrate, amino acids such as proline and arginine), by repressing the transcription of some genes (NCR-sensitive genes) involved in the utilization (transport : permeases ; degradation : catabolic enzymes) of the poorer nitrogen sources (Cooper and Sumrada, precited). Under nitrogen-rich conditions (repressive conditions), Ure2p forms complexes with Gln3p and Gat1p, transcriptional activators of the GATA family, in the cytoplasm, to decrease the transcription of genes encoding the proteins needed to transport and degrade poor nitrogen sources (NCR-sensitive expression; Ter Schure *et al.,* precited; Scherens et al., FEMS Yeast Res., 2006, 6, 777-791). On the other hand, when only non-preferred nitrogen sources are available, Gln3p and Gat1p are dephosphorylated, and move from the cytoplasm to the nucleus, where they positively regulate the expression of NCR-sensitive genes for nitrogen transport and catabolism. Nitrogen Catabolic Repression control is effective during wine fermentation (Rossignol et al., Yeast, 2003, 20, 1369-1385; Beltran et al., FEMS Yeast Res., 2004, 4, 625-632) and can be removed by deleting the *URE2* gene. Such deletion improves nitrogen uptake leading to an improvement in fermentation kinetics under enological conditions (Salmon, J.M. and Barre P., Applied and Environ. Microbiol., 1998, 64, 3831-3837).

Ure2p displays a GST fold containing an N-terminal thioredoxin-fold domain and a C-terminal alpha helical domain. A transmissible conformational change of Ure2p results in a prion called [Ure3], an inactive, self-propagating and infectious amyloid. The N-terminal thioredoxin-fold domain (N-terminal ~ 90 amino acids) is sufficient to induce the [Ure3] phenotype and is also called the prion domain of Ure2p (Masison et al., P.N.A.S., 1997, 94, 12503-12508). In addition to its role in nitrogen regulation, Ure2p confers protection to cells against heavy metal ion and oxidant toxicity, and shows glutathione (GSH) peroxidase activity (Bai et al., J. Biol. Chem., 2004, 279, 50025-50030). The GST active site is located in a cleft between the N- and C-terminal domains.

The *URE2* gene that is conserved in yeast encodes the Ure2p-like subfamily of proteins composed of the *Saccharomyces cerevisiae* Ure2p of the amino acid sequence SEQ ID NO : 1 (354 amino acids, Accession number NP_014170.1) and related Glutahione S-Tranferases (GSTs) of other yeasts having a sequence that is homologous to SEQ ID NO : 1. For example, the *Saccharomyces cerevisiae URE2* gene is located from positions 219138 to 220202 on the complement strand of chromosome XIV (Accession number NC_001146; gene ID 855492, locus tag YNL229C). The *URE2* gene sequence of other yeasts is available on the databases.

The inventors have constructed a *URE2* gene deletion mutant (*Δure2* mutant) in a wine yeast strain background and shown that, under enological conditions, the Δ*ure2* mutant releases 3 times as many volatile thiols as the wild type strain. Surprisingly, the 3MH (*R*)/(*S*) balance was increased significantly in the *Δure2* strain, putting the (*R*)/(*S*) ratio at 70:30, as compared to 50:50 in the wild type strain. These results indicate that, in addition to the enhancement of aroma release, *Δure2* deletion modifies the sensory perception of 3MH aroma of wine by intensifying the grapefruit overtones (3MH(*R*) descriptor).

In addition, contrary to what was suggested from previous reports (Howell *et al.,* 2005, precited), *BNA3* deletion experiments in a wine yeast background, indicated that the *BNA3* gene does seem not play a pivotal role in the release of volatile thiols, under enological conditions.

Thus, from a biotechnological point of view, these novel properties of *URE2* mutant yeasts open new avenues for consistently producing wine to definable specifications and style, as well as for developing wine yeast starter strains with optimized volatile thiol release capabilities.

The invention relates to the use of an *URE2* mutant yeast *(URE2* mutant) having a loss-of-function mutation in the *URE2* gene for increasing the release of aromatic volatile thiol by yeast during fermentation.

### . Definition

- "mutation" refers to the substitution, deletion, addition of one or more nucleotides in a polynucleotide (cDNA, gene).
- *"**URE2* mutant having a loss-of-function mutation in the *URE2* gene", refers to a yeast mutant which has a partial or total (null mutation) loss of function in the *URE2* gene and exhibits defects in nitrogen catabolite repression (NCR) and/or in glutathione peroxidase activity. The defect in NCR results in a mutant that is able to assimilate poorer nitrogen sources in the presence of good nitrogen sources. The defect in glutathione peroxidase activity increases the sensitivity to heavy metal ions and oxidants.
- by "homologous" is intended a sequence with enough identity to another one to lead to a protein having the same function, particularly having at least 70 % identity, preferably 80 % identity and more preferably 90 %.
- "Identity" refers to sequence identity between two nucleic acid molecules or polypeptides. Identity can be determined by comparing a position in each sequence which may be aligned for purposes of comparison. When a position in the compared sequence is occupied by the same base, then the molecules are identical at that position. A degree of similarity or identity between nucleic acid or amino acid sequences is a function of the number of identical or matching nucleotides at positions shared by the nucleic acid sequences. Various alignment algorithms and/or programs may be used to calculate the identity between two sequences, including FASTA, or BLAST which are available as a part of the GCG sequence analysis package (University of Wisconsin, Madison, Wis.), and can be used with, e.g., default settings.
- "wine yeast" refers to a yeast, usually a strain of *Saccharomyces sp.,* with high ethanol tolerance, resistance to sulfur dioxide and ability to rapidly and efficiently ferment grape juice containing 20 to 25 % sugar without producing organoleptic defects (acetate (volatile acidity)), hydrogen sulfide and phenol compounds).
- "aromatic volatile thiol" refers to an organic chemical compound that: (i) contains the functional group composed of a sulfur atom and a hydrogen atom (-SH), (ii) has high enough vapour pressures under normal conditions to significantly vaporize, and (iii) is odorous (flavour active).
- "fermentation" refers to any of a group of chemical reactions in which a living or non-living microorganism causes an organic substance to break down into simpler substances; especially, the anaerobic breakdown of sugar into alcohol (alcoholic fermentation).
- wine refers to an alcoholic beverage produced by the fermentation of the juice of fruits, usually grapes. Non-grape wines are called fruit wine or country wine. Other products made from starch based materials, such as barley wine, rice wine (sake), are more similar to beers. Beverages made from other fermentable material such as honey (mead), or that are distilled, such as brandy, are not wines.
- beer refers to an alcoholic fermented beverage most often made from malted barley, hops, yeast and water.

According to the present invention, the volatile thiol is released from non-volatile thiol precursor(s) present in a product (initial product), during fermentation by the *URE2* mutant. Fermentation of the initial product by the *URE2* mutant results in a fermented product (finished product), in particular an alcoholic beverage (wine, cider, beer), having improved aroma as a result of higher aroma active volatile thiol content.

According to the present invention, the improvement of the aroma includes the enhancement of the aroma resulting from the enhanced release of the different nuances of said aroma. It may also include the selective enhancement of a specific nuance of the aroma, resulting in a selective modification of the aroma.

The *URE2* mutant may be a natural mutant or a mutant obtained by genetic manipulation.

The mutant is derived from yeast strains able to ferment high amount of sugar including wine, brewery, bakery and distillation strains and/or any other strains well known for their ability to release aromatic compounds.

Natural mutants lacking Ure2p function (null alleles or null mutation) are well-known in the art ; for example *URE2* mutant alleles, also known as *usu* and *gdh*CR, have been described previously (Grenson, M., Eur. J. Biochem., 1969, 11, 249-260 ; Grenson et al., Mol. Gen. Genet., 1974, 128, 73-85 ; Courchesne et al., J. Bacteriol., 1988, 170, 708-713 ; Drillien et al., J. Bacteriol., 1972, 109, 203-208 (FL467-3D strain) ; Legrain et al., Eur. J. Biochem., 1982, 123, 611-616 (12597a strain); Brandriss et al., Can. J. Bot., 1995, 73 (Suppl.) : S153-S159 ; Coffman et al., J. Bacteriol., 1994, 176, 7476-7483 ; Xu et al., Mol. Cell. Biol., 1995, 15, 2321-2330; Coschigano, P.W. and B. Magasanik, Mol. Cell. Biol., 1991; 11, 822-832). These mutants were characterized as possessing: (i) nitrogen catabolic enzymes insensitive to nitrogen catabolite repression, mainly in the pathways involved in glutamate, glutamine, arginine, allantoin, urea, gamma-aminobutyrate and proline assimilation, (ii) increased amino acid permeases activity, (iii) resistance of ureidosuccinate transport to nitrogen repression. The *URE2* mutation was identified for some of the mutants. For example, the *ure2-1* mutation identified in the P5-5D strain of *S. cerevisiae* is a t to c transition at position 1119 of the nucleotide sequence causing the phenylalanine at position 313 of the amino acid sequence to be replaced by a serine (Coschigano, P.W. and Magasanik B., precited).

Other *URE2* mutants may be obtained by screening for yeasts having a phenotype as defined above.

*URE2* mutants having large deletions in the *URE2* coding sequence and lacking Ure2p function, have been constructed according to standard gene disruption techniques in yeast (Rothstein, R.J., Methods Enzymol., 1983, 101, 202-211 ; Wach et al., Yeast, 1994, 10, 1793-1808). For example, *URE2* disruption mutants were constructed by replacing one chromosomal copy of the *URE2* coding sequence, entirely or in part (*Sac*II*-Pvu*II or *Hind*III fragments), by a complementing selection marker for yeast *(LEU2, URA3).*

*URE2* mutants were constructed by random mutagenesis (UV mutagenesis) and screening for yeasts derepressed for proline utilization under nitrogen repression, able to grow in the presence of proline as the sole nitrogen source and methylamine (ammonium analog) at repressive concentration (Salmon, J.M. and Barre P., Applied and Environmental Microbiology, 1998, 64, 3831-3837).

Other mutants may be constructed by standard mutagenesis (random and site-directed) techniques which are well-known in the art, followed by screening for *URE2* mutants having a phenotype as defined above.

The *URE2* mutant may be homozygous or heterozygous for the *URE2* mutation.

In addition, the *URE2* mutation may be introduced in an appropriate background by spore to spore mating and segregation analysis using conventional yeast genetic methods using homothallic or heterothallic strains (US Patent 6,140,108; Marullo et al., FEMS Yeast Res., 2006, 6, 268-279; Bakalinsky et al., Applied and Environ. Microbiology, 1990, 56, 849-857).

The release of volatile thiol is measured by an appropiate method, well-known in the art, such as for example : specific extraction of volatile thiols (Tominaga et al., J. Agric. Food. Chem., 1998, 46, 1044-1048; Tominaga et al., J. Agric. Food. Chem., 2000, 48, 1799-1802), followed by gas chromatography coupled with mass spectrometry (GC/MS; Tominaga et al., J. Agric. Food. Chem., 1998, 46, 1044-1048), flame photometric detection (GC/FPD; Tominaga, T. and Dubourdieu, D., Flavour Fragrance J., 1997, 12, 373-376), olfactometry (GC/olfactometry; Darriet et al., J. Int. Sci. Vigne Vin., 1991, 25, 167-174) or atomic emission detection (GC/AED; Howell et al., FEMS Microbiology Letters, 2004, 240, 125-129).

The increase of volatile thiol by the *URE2* mutant may be assessed by comparison with an isogenic yeast strain not mutated in the *URE2* gene, grown in the same conditions.

According to a preferred embodiment of said use, the *URE2* mutant is a null mutant lacking Ure2p function.

According to another preferred embodiment of said use, the URE2 mutant yeast is homozygous for the *URE2* mutation.

According to another preferred embodiment of said use, the *URE2* mutant is a mutant of *Saccharomyces sp.* or of a derived interspecific hybrid. In particular, the mutant may be derived from *S. cerevisiae, S. bayanus (var. uvarum), S. paradoxus* or from interspecific hybrids of *Saccharomyces,* such as *S. pastorianus.*

According to another preferred embodiment of said use, the *URE2* mutant is a mutant of a wine yeast, preferably of a commercial wine yeast strain chosen from:VL3c, X5 and RX60.

According to another preferred embodiment of said use, the volatile thiol derives from a S-cysteine conjugate precursor, as indicated in Table I

**Table I: Volatile thiols and their precursor**

| **Volatile thiol** | **Precursor** |
|---|---|
| 4-mercapto-4-methylpentan-2-one (4MMP) | S-4-(4-methylpentan-2-one)-L-cysteine |
| 4-mercapto-4-methylpentan-2-ol (4MMPOH) | S-4-(4-methylpentan-2-ol)-L-cysteine |
| 3-mercaptohexan-1-ol (3MH) | S-3-(hexan-1-ol)-L-cysteine |
| 3-mercaptohexyl acetate (A3MH or 3MHA) | 3MH* |
| 3-mercaptopentan-1-ol (3MPOH) | S-3-(pentan-1-ol)-L-cysteine |
| 3-mercaptoheptan-1-ol (3MHepOH) | S-3-(heptan-1-ol)-L-cysteine |
| 3-mercapto-2-methylbutan-1-ol (3M2MBOH) | S-3-(2-methylbutan-2-ol)-L-cysteine |
| 3-mercapto-2-methylpentan-1-ol (3M2MPOH) | S-3-(2-methylpentan-2-ol)-L-cysteine |

| | |
|---|---|
| *A3MH is the product of 3MH esterification | |

The volatile thiol is preferably selected from the group consisting of: 4MMP, 3MH, 4MMPOH, 3MHA, 3MPOH, 3MHepOH, 3M2MBOH, and/or 3M2MPOH, more preferably from 4MMP, 3MH and/or 3MHA.

Furthermore, the 3MH which is released by the yeast mutant may advantageously consist of more (*R*) enantiomer than (*S*) enantiomer; the 3MH consists advantageously of at least 70 % of (*R*) enantiomer ((*R*) to (*S*) enantiomer ratio superior or equal to 70:30).

According to another preferred embodiment of said use, the release of at least two different aromatic volatile thiols is increased during fermentation.

According to another preferred embodiment of said use, the release of aromatic volatile thiol is increased at least by a factor of 3.

According to another preferred embodiment of said use, said fermentation is alcoholic fermentation, preferably wine fermentation.

The invention relates also to a method for enhancing the aroma of a fermented product produced by fermentation of an initial product containing at least one non-volatile thiol precursor, comprising the incubation of the initial product with *a URE2* mutant yeast as defined above, under conditions allowing fermentation by the *URE2* mutant to occur, and thereby increase the release of the volatile thiol(s)-from the non-volatile thiol precursor(s)-, by the mutant yeast, during fermentation.

According to a preferred embodiment of said method the fermented product is an alcoholic beverage, preferably wine, more preferably wine made from a simple or non-floral grape variety, such as for example: Sauvignon Blanc, Gewürztraminer, Riesling, Semillon, Colombard, Scheurebe, Petit Manseng, Cabernet Sauvignon and Merlot.

According to another preferred embodiment of said method the non-volatile thiol precursor is a S-cysteine conjugate precursor.

The volatile thiol is preferably selected from the group consisting of: 4MMP, 3MH, 4MMPOH, 3MHA, 3MPOH, 3MHepOH, 3M2MBOH, and/or 3M2MPOH, more preferably from 4MMP, 3MH and/or 3MHA.

According to another preferred embodiment of said method, the release of aromatic volatile thiol(s) is increased at least by a factor of 3.

The invention relates also to a method for enhancing the grapefruit overtone of a fermented product produced by fermentation of an initial product containing S-3-(hexan-1-ol)-L-cysteine (3MH precursor), comprising the incubation of the initial product with a *URE2* mutant yeast as defined above, under conditions allowing fermentation by the *URE2* mutant to occur, and thereby increase the release of the 3MH (R) enantiomer, by the mutant yeast, during fermentation.

According to a preferred embodiment of said method, the fermented product is an alcoholic beverage, preferably wine, more preferably wine made from a simple or non-floral grape variety, such as for example: Sauvignon Blanc, Gewürztraminer, Riesling, Semillon, Colombard, Scheurebe, Petit Manseng, Cabernet Sauvignon and Merlot.

According to another preferred embodiment of said method, the 3MH *(R)* to *(S)* enantiomer ratio is superior or equal to 70:30 (3MH consisting of at least 70 % of *(R)* enantiomer)

The invention relates also to a method of making a yeast strain able to improve the aroma of a fermented product, comprising at least the steps of:
(a) preparing *URE2* mutant(s) from an initial yeast (parent yeast), by any appropriate means,
(b) incubating the *URE2* mutant(s) of step (a) in a fermentation medium containing non-volatile thiol precursor(s), under conditions allowing fermentation by the *URE2* mutant to occur, and thereby release of the volatile thiol(s)-from the non-volatile thiol precursor(s)-, by the mutant yeast, during fermentation,
(c) measuring the volatile thiol(s) released in the fermented medium, by any appropriate means, as defined above, and
(d) identifying the *URE2* mutant(s) able to release high level aromatic volatile thiol, as compared to the initial yeast (parent yeast).

The parent yeast is a yeast strain able to ferment high amount of sugar including wine, brewery, bakery and distillation strains and/or any other strain well known for its ability to release aromatic compounds.

The mutants of step (a) are prepared by genetic manipulation, according to standard methods, as defined above.

Preferably, the *URE2* mutant releases at least twice, preferably at least three times more volatile thiol than the initial yeast (parent yeast).

According to a preferred embodiment of said method, the parent yeast is a strain of *Saccharomyces sp.* or of a derived interspecific hybrid, as defined above

According to another preferred embodiment of said method, the parent yeast is a wine yeast strain, preferably chosen from : VL3c, X5 and RX60.

According to another preferred embodiment of said method, the fermentation of step (b) is alcoholic fermentation.

According to another preferred embodiment of said method, step (c) comprises the measurement of volatile thiol(s) selected from the group consisting of: 4MMP, 3MH, 4MMPOH, 3MHA, 3MPOH, 3MHepOH, 3M2MBOH, and/or 3M2MPOH, more preferably from 4MMP, 3MH and/or 3MHA.

The invention relates also to a method for screening yeasts able to improve the aroma of a fermented product, comprising at least the step of:
(a) providing a collection of yeasts,
(b) identifying yeasts having a mutation in the *URE2* gene, by any appropriate means,
(c) incubating the *URE2* mutant(s) of step (b) in a fermentation medium containing non-volatile thiol precursor(s), under conditions allowing fermentation by the *URE2* mutant to occur, and thereby release of the volatile thiol(s)-from the non-volatile thiol precursor(s)-, by the mutant yeast, during fermentation,
(d) measuring the volatile thiol(s) released in the fermented medium, by any appropriate means, and
(e) identifying the *URE2* mutant(s) able to release high level volatile thiol, as compared to yeast having a wild-type *URE2* gene.

The collection of yeasts (step (a)) is a collection of yeast strains able to ferment high amount of sugar including wine, brewery, bakery and distillation strains and/or any other strains well known for its ability to release aromatic compounds, as defined above.

Preferably, the *URE2* mutant releases at least twice, preferably at least three times more volatile thiol than a yeast having a wild-type *URE2* gene in the same genetic background.

According to another preferred embodiment of said method, the fermentation of step (c) is alcoholic fermentation.

According to another preferred embodiment of said method, step (d) comprises the measurement of volatile thiol(s) selected from the group consisting of: 4MMP, 3MH, 4MMPOH, 3MHA, 3MPOH, 3MHepOH, 3M2MBOH, and/or 3M2MPOH, more preferably from 4MMP, 3MH and/or 3MHA.

The practice of the present invention will employ, unless otherwise indicated, conventional techniques of microbiology, recombinant DNA, molecular biology, cell biology and cell culture which are within the skill of the art. Such techniques are explained fully in the literature. See, for example, Methods of classical genetics. Biotechnology Handbooks Saccharomyces. Vol 4. (Wickner, R, Truite M. & Oliver S., Eds, 1991, pp 101-147); Current Protocols in Molecular Biology (Frederick M. AUSUBEL, 2000, Wiley and son Inc, Library of Congress, USA); Molecular Cloning: A Laboratory Manual, Third Edition, (Sambrook et al, 2001, Cold Spring Harbor, New York: Cold Spring Harbor Laboratory Press).

In addition to the preceding features, the invention further comprises other features which will emerge from the description which follows, which refers to examples illustrating the use of *URE2* mutants according to the invention, as well as to the appended drawing in which:

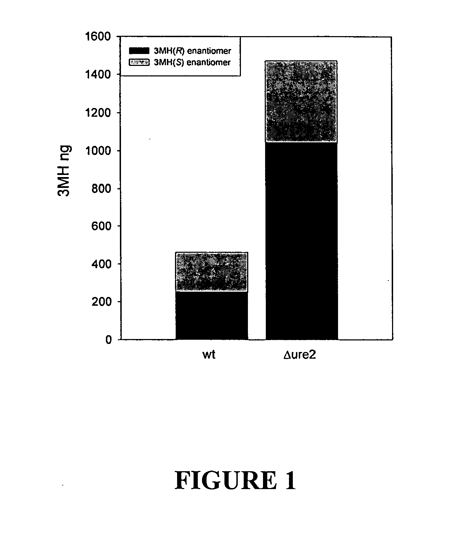

- figure 1 illustrates the stereo-selectivity of *Δure2* mutants for 3MH enantiomers. In the *Δure2* deletion strain, 3MH production was strongly increased compared to control; moreover, the 3MH(*R*)/(*S*) ratio was largely unbalanced, with 70% of 3MH(R). Values are the average of triplicate experiments.

### Example 1: URE2 deletion improves thiol release under enological conditions

### 1) Material and methods

### a) Culture conditions and fermentation

Yeasts were grown at 30°C on complete YPD medium (1 % yeast extract, 1 % peptone, and 2 % dextrose) solidified with 2 % agar if necessary. G418 (100 µg ml⁻¹) was added to YPD to select transformed strains. Sporulation was induced on acetate medium (1 % potassium acetate, 2 % agar) for three days at 24°C. Fermentation experiments were carried out at 24°C in 350 ml-bottles containing a synthetic model medium previously described as KP medium (Marullo et al., FEMS Yeast Research, 2006, 6, 268-279). This medium was buffered to pH 3.3 and contained 80 g L⁻¹ glucose, 80 g L⁻¹ fructose, and 190 mg L⁻¹ available nitrogen. Aroma precursors (P-4MMP and P-3MH) were added to the medium before yeast inoculation at different concentrations, as indicated in the text. Yeast strains were inoculated at 1.10⁶ cells ml⁻¹ from an overnight pre-culture. Volatile thiol release was measured after complete fermentation (measured by weight loss). All experiments were carried out in triplicate and independently repeated two or three times.

### b) Yeast strains

*Saccharomyces cerevisiae* strain VL3-1D was derived from a commercial starter (VL3c, Laffort OEnologie). VL3-1D, a homothallic diploid spore clone (*HO*/*HO*) obtained by tetrad micro-dissection, is assumed to be totally homozygous (Marullo et al., FEMS Yeast Research, 2004, 4, 711-719). The Δ-U (*Δure2*/*Δure2*) and Δ-B (*Δbna3*/*Δbna3*) strains were constructed from the VL3-1D strain using PCR deletion strategy (Baudin et al., Nucleic Acids Research, 1993, 21, 3329-330; Wach et al., Yeast, 1994, 10, 1793-1808). Deletion cassettes were obtained by amplifying the genomic DNA of appropriate deleted strains (BY4742 background) from the Euroscarf collection (Frankfurt, Germany). The *ure2::KanMx4* and *bna3::KanMx4* cassettes were obtained using p59: 5'-caaatgccgagaaaaataccgc-3' (SEQ ID NO: 2) and p60: 5'-aaacgaacgccgaaacacata-3'(SEQ ID NO: 3), p1: 5'-gagcagattgttttgagtagg-3'(SEQ ID NO: 4), p2: 5'-ttcctaagcaactcatcgtg-3' (SEQ ID NO: 5) primer pairs, respectively. Cells were chemically transformed (Puig et al., J. Agric. Food Chem., 1998, 46, 1689-1693) and positively selected on YPD-G418. G418-growing clones were sporulated and micro-dissected in order to obtain fully homozygous spore clones carrying two deleted gene copies. Correct deletions were verified by PCR.

### c) Precursor synthesis

### P-4MMP synthesis

*S*-4-(4-methylpentan-2-one)-L-cysteine (P-4MMP) was synthesized by the addition of mesityl oxide (22 mmol) in L-cysteine (20 mmol) in distilled water for 16 h. After extraction with diethyl ether (2 x 30 ml), the aqueous layer was cooled to 0°C and di-*tert*-butyl dicarbonate (4.6 g, 21 mmol) in 20 ml of THF was added dropwise and stirred overnight. After elimination of THF by rotary evaporation, the aqueous layer was adjusted to pH 2-3 with 10 % citric acid in water and extracted with EtOAc (3 x 100 ml). Combined organic layers were washed with water (50 ml) and brine (50 ml), dried over magnesium sulfate, filtered, and concentrated under reduced pressure. The purification of the crude product by silica gel chromatography (pentane/diethyl ether - 35/65, Rf = 0.5) gave a colorless oil (4.5 g, 70 %). 1 ml of aquous HCl 10M (10 mmol) was added to a *N*-(*tert*-Butoxycarbonyl)-*S*-4-(4-methylpentan-2-one)-L-cysteine (0.63g, 2 mmol) at room temperature. After reaction was complete, excess HCl was removed by heating at 60°C under reduced pressure (0.1 mm Hg).

### P-3MH synthesis

S-3-(hexan-1-ol)-L-cysteine (P-3MH) was synthesized according to (Wakabayashi et al., J. Agric. Food Chem., 2004, 52, 110-116) and modified as follows: *Trans*-2-hexen-1-al (5 mmol) was added to a stirred solution of N*-*(*tert-*Butoxycarbonyl)-L-cysteine (4.5 mmol) and cesium carbonate (2.25 mmol) in acetonitrile overnight. After elimination of acetonitrile by rotary evaporation, sodium borohydride (2.5 mmol) in 10 ml of methanol was added dropwise and stirred overnight. The deprotection of 0.5 ml of N-(*tert*-Butoxycarbonyl)-*S*-3-(hexan-1-ol)-L-cysteine was carried out by the addition of HCl 10M (5 mmol). The crude product was concentrated and purified on an ion exchange column (DOWEX 50 WX8).

### d) Volatile thiol assays

Volatile thiols were extracted at the end of fermentation from 250 ml of synthetic medium using the previously described method (Tominaga et al., J. Agric. Food Chem., 1998b, 46, 1044-1048) and modified according to (Tominaga et al., J. Agric. Food Chem., 2000b, 48, 1799-1802). Amounts of 4MMP, 3MH and 3MHA were quantified by GC-MS (Gas chromatography and solid-phase microextraction) according to methods described by Tominaga *et al.,* 1998b.

### 2) Results

The VL3-1D strain is a (*HO*/*HO*) meiotic spore clone of the commercial strain VL3c previously described for its strong capacity to reveal volatile thiols (Murat et al., Am. J. Enol. Vitic., 2001, 52, 136-140; Howell et al., FEMS Microbiol Lett., 2004, 240, 125-129; Howell et al., Appl. Environ. Microbiol., 2005, 71, 5420-5426; Masneuf-Pomarède et al., Int. J. Food Microbiol., 2006, 108385-390). The Δ-U strain carrying two deleted copies of *URE2* (Δ*ure2*/Δ*ure2*) was obtained from VL3-1D by *ure2::KanMx4* cassette mediated deletion.

The kinetic properties of this mutant were compared to the *wt* strain using bioreactors as previously described (Marullo et al., FEMS Yeast Research, 2006, 6, 268-279). Under enological conditions, both strains showed identical kinetic parameters.

Volatile thiol release of *Δure2* and *wt* strains was then assayed in 350 ml-batch cultures using a synthetic grape medium with the addition of synthetic precursors. Precursor concentrations were adjusted to 20 nM for P- 4MMP and 2000 nM for P-3MH in accordance with standard Sauvignon Blanc must composition (Peyrot des Gachons et al., J. Agric. Food Chem., 2000, 48, 3387-3391). During fermentation, yeast metabolism produced 4-mercapto-4-methylpentan-2-one (4MMP) from its *S*-cysteine conjugate (*S*-4-(4-methylpentan-2-one)-L-cysteine) henceforth referred to as P-4MMP. Similarly, 3-mercaptohexanol (3MH) was produced from *S-*3((hexan-1-ol)-L-cysteine (henceforth referred to as P- 3MH). A part of 3MH can also be acetylated during fermentation, producing 3-mercaptohexyl acetate (3MHA). At the end of the fermentation, 4MMP, 3MH, and 3MHA produced by yeast were organically extracted and measured by GC-MS (Tominaga et al., Flavour Fragr. J., 1998a, 13, 159-162). The absence of volatile thiols in unfermented control was previously demonstrated (Murat et al., Am. J. Enol. Vitic., 2001, 52, 136-140). As shown in Table II, *Δure2* mutant released 3 times as many volatile thiols (4MMP, 3MH, and 3MHA) as the wild type strain.

**Table II: URE2-deletion enhances volatile thiol release under enological conditions**

| Strains | Aromas released (ng L⁻¹)^{a} | | |
|---|---|---|---|
| | 4MMP | 3MH | 3MHA |
| VL3-1 D (wt) | 30.2 _{+/- 1.9} | 467.5 _{+/- 55.4} | 85.9 _{+/-21.9} |
| Δ-U (Δure2) | 87.6* _{+/- 8.5} | 1473.2* _{+/- 147.2} | 276.2* _{+/-14.5} |

| | | | |
|---|---|---|---|
| ^{a} Aromas were measured at the end of alcoholic fermentation of synthetic medium containing aroma precursors: P-4MMP=20 nM, P-3MH 2000 nM. Values are the means of two independent experiments carried out in triplicate. * Significantly different from control, ANOVA p value < 0.001 | | | |

### Example 2: URE2 deletion modifies 3MH enantiomer balance improves

### 1) Material and methods

### Separation of two 3MH enantiomers

Extracted volatile thiols were injected into a Lipodex C (50 m × 0.25 mm) chiral column (INTERCHIM) to separate the two 3MH enantiomers. Chromatography conditions were identical to those described by Tominaga et al. (J. Agric. Food Chem., 2006, 54, 7251-7255).

### 2) Results

3MH is a chiral molecule with 2 enantiomers, 3MH(*R*) and 3MH(*S*). Using a specific GC-MS methodology (Tominaga et al., J. Agric. Food. Chem., 2006, 54, 7251-7255), R and S-3MH were measured in synthetic medium at the end of alcoholic fermentation. The relative amount of 3MH enantiomers of VL3-1D (wt) and Δ-U (*Δure2*) strains are shown in Figure 1. As generally observed in dry white wine, the medium fermented by VL3-1D (*wt*) presented a racemic mix between 3MH (*R*) and (*S*) enantiomers (50:50). Surprisingly, the (*R*) and (*S*)-forms in the *Δure2 strain* increased significantly (4.1 times and 2.0 times respectively), putting the (*R*)/(*S*) ratio at 70:30. This 3MH (*R*)/(*S*) balance modification suggests that stereo-selective mechanisms occurred in *Δure2* mutants. These findings indicated that, in addition to the enhancement of aroma release, *Δure2* deletion might modify the sensory perception of 3MH aroma of wine by intensifying the grapefruit overtones (3MH(*R*) descriptor).

These findings demonstrate that the *URE2* gene strongly inhibits the volatile thiol liberation mechanisms during alcoholic fermentation, probably involving the Nitrogen Catabolic Repression. Due to the upstream regulation of *URE2,* several regulated targets may affect thiol release. The full activation of Gln3p and Gatlp transcription factors may enhance the expression of two main gene categories. First, up-regulated genes encoding nitrogen catabolic enzymes (Scherens et al., FEMS, Yeast Research, 2006, 6, 777-791) may contribute to enhancing the pool of precursor cleaving enzymes. Second, expressed poor-nitrogen transporters may increase precursor uptake due to its amino acid-like structure.

### Example 3: Analysis of Δbna3 mutant under enological conditions

### 1) Material and methods

### see example 1

### 2) Results

Among the candidate genes of *S. cerevisiae* encoding putative *β-*lyase enzyme, *BNA3* encoding an arylformamidase was found to contribute strongly to volatile thiol release. The *BNA3* effect was previously assayed in a synthetic medium containing 0.1 g L⁻¹ (450 µM) of P-4MMP (Howell et al., Appl. Environ. Microbiol., 2005, 71, 5420-5426). However, in natural grape juice only a few µg L⁻¹ are found (0.5 to 20 nM; Peyrot des Gachons et al., J. Agric. Food Chem., 2000, 48, 3387-3391).

Therefore the *BNA3* gene was deleted in VL3-1D background, producing the Δ-B strain (*Δbna3*/*Δbna3*). Then, the deletion effect on 4MMP release after alcoholic fermentation, was assayed for *wt* and *Δbna3* strains, in synthetic model medium containing 20 nM, 20,000 nM, 200,000 nM of P-4MMP, corresponding respectively to 1, 1,000, and 10,000 times the P-4MMP concentration in grape juice (Table III).

**Table III: BNA3 deletion effect is dependent on initial P-4MMP concentration**

| Strains | 4MMP released (ng L⁻¹)^{a} | | |
|---|---|---|---|
| | P-4MMP added (X folds must concentrations)^{b} | | |
| | 10,000 X (n=3) | 1,000 X (n=2) | 1 X (n=3) |
| VL3-1D (*wt*) | 44.2 10⁴ _{+/-2.9 10 4} | 23.2 10³ _{+/-10.2 10³} | 29.2 _{+/-9.2} |
| Δ-B (*Δbna3*) | 8.7 10⁴ * _{1.2 10⁴} | 26.5 10³ _{+/-3.8 10³} | 41.3_{+/-3.2} |

| | | | |
|---|---|---|---|
| ^{a} Values are means of (n) independent experiments carried out in triplicate ^{b} Various P-4MMP concentrations were used (10, 000 X, 1,000X and 1 X) corresponding to 200,000, 20,000, and 20 nM, respectively. * Significantly different from control, ANOVA p value <0.05 | | | |

At a concentration of 10,000 X, with the *BNA3* deletion, the 4MMP release at the end of the fermentation dropped by 81% in accordance with Howell's findings. This result confirmed the probable β-lyase activity of Bna3p and its involvement in the cleavage of P-4MMP in aroma.

Surprisingly, when decreasing the amount of P-4MMP to 1000 X or 1 X, *BNA3* deletion no longer affected aroma release. Moreover, *BNA3* deletion did not influence the production of two other aromas (3MH and 3MHA) when P-3MH concentration corresponded to grape juice (2,000 nM). All these findings suggested that, under enological conditions *(i.e.* grape juice P-4MMP concentration), the native *BNA3* gene inhibits 4MMP release, while at higher concentrations it seems to catalyze its liberation. Like in Howell *et al*, the *BNA3* deletion effect was studied using a spore clone derived from the same commercial starter, VL3c (Laffort Oenologie, France). Thus, the starting genetic material came from the same genetic background, sharing more than 50 % of genome identity.

These results could be explained, by making the hypothesis that Bna3p shows a weak affinity for precursors compromising the cleavage of a few nM of P-4MMP. More generally, these findings suggest that: (*i*) other enzymes having a very high affinity for aroma precursors have yet to be discovered, (*ii*) aroma conversion is achieved by a wide pool of cytosolic enzymes able to carry out β-substitution on an amino acid skeleton.

## Claims

1. Use of a *URE2* mutant yeast having a loss-of-function mutation in the *URE2* gene for increasing the release of aromatic volatile thiol by yeast during fermentation.

2. The use of claim 1, wherein the mutant is a null mutant lacking Ure2p function.

3. The use of claim 1 or claim 2, wherein the mutant is a mutant of *Saccharomyces sp* or of a derived interspecific hybrid

4. The use of anyone of claims 1 to 3, wherein the mutant is a mutant of a wine yeast.

5. The use of anyone of claims 1 to 4, wherein the aromatic volatile thiol derives from a S-cysteine conjugate precursor.

6. The use of anyone of claims 1 to 5, wherein the volatile thiol is selected from the group consisting of: 4MMP, 3MH, 4MMPOH, 3MHA, 3MPOH, 3MHepOH, 3M2MBOH, and/or 3M2MPOH.

7. The use of claim 6, wherein the 3MH which is released by the mutant consists of more *(R)* enantiomer than *(S)* enantiomer.

8. The use of claim 7, wherein the 3MH consists of at least 70 % of (R) enantiomer.

9. The use of anyone of claims 1 to 8, wherein the release of volatile thiol is increased at least by a factor of 3.

10. The use of anyone of claims 1 to 9, wherein the fermentation is alcoholic fermentation of wine.

11. A method for enhancing the aroma of a fermented product produced by fermentation of an initial product containing at least one non-volatile thiol precursor, comprising the incubation of the initial product with a *URE2* mutant yeast as defined in anyone of claims 1 to 4, under conditions allowing fermentation by the *URE2* mutant to occur, and thereby increase the release of the volatile thiol(s) by the mutant yeast, during fermentation.

12. The method of claim 11, wherein the non-volatile thiol precursor is a S-cysteine conjugate precursor.

13. The method of claim 11 or claim 12, wherein the aromatic volatile thiol is selected from the group consisting of: 4MMP, 3MH, 4MMPOH, 3MHA, 3MPOH, 3MHepOH, 3M2MBOH, and/or 3M2MPOH.

14. The method of anyone of claims 11 to 13, wherein the release of aromatic volatile thiol is increased at least by a factor of 3.

15. A method for enhancing the grapefruit overtone of a fermented product produced by fermentation of an initial product containing S-3-(hexan-1-ol)-L-cysteine (3MH precursor), comprising the incubation of the initial product with a *URE2* mutant yeast as defined in anyone of claims 1 to 4, under conditions allowing fermentation by the *URE2* mutant to occur, and thereby increase the release of the 3MH (R) enantiomer, by the mutant yeast, during fermentation.

16. The method of claim 15, wherein the 3MH (R) and (*S*) enantiomer ratio is superior or equal to 70:30.

17. The method of anyone of claims 11 to 16, wherein the fermented product is an alcoholic beverage.

18. The method of claim 17, wherein the alcoholic beverage is wine.

19. The method of claim 18, wherein the wine is made by fermentation of a simple or non-floral grape variety selected from the group consisting of: Sauvignon Blanc, Gewürztraminer, Riesling, Semillon, Colombard, Scheurebe, Petit Manseng, Cabernet Sauvignon and Merlot.

20. A method of making a yeast strain able to improve the aroma of a fermented product, comprising at least the steps of:
(a) preparing *URE2* mutant(s) from an initial yeast, by any appropriate means,
(b) incubating the *URE2* mutant(s) of step a) in a fermentation medium containing non-volatile thiol precursor(s), under conditions allowing fermentation by the *URE2* mutant(s) to occur, and thereby release of the volatile thiol(s), by the mutant, during fermentation,
(c) measuring the volatile thiol(s) released in the fermented medium, by any appropriate means, and
(d) identifying the *URE2* mutant(s) able to release high level aromatic volatile thiol, as compared to the initial yeast.

21. The method of claim 20, wherein the initial yeast in (a) is a strain of *Saccharomyces sp* or of a derived interspecific hybrid.

22. The method of claim 20 or claim 21, wherein the initial yeast in (a) is a wine yeast strain.

23. The method of claim 22, wherein the wine yeast strain is selected from the group consisting of: VL3c, X5 and RX60.

24. The method of anyone of claims 20 to 23, wherein the fermentation of step (b) is alcoholic fermentation.

25. The method of anyone of claims 20 to 24, wherein step (c) comprises the measurement of volatile thiol(s) selected from the group consisting of: 4MMP, 3MH, 4MMPOH, 3MHA, 3MPOH, 3MHepOH, 3M2MBOH, and/or 3M2MPOH.

26. A method for screening yeasts able to improve the aroma of a fermented product, comprising at least the step of:
(a) providing a collection of yeasts,
(b) identifying yeasts having a mutation in the *URE2* gene, by any appropriate means,
(c) incubating the *URE2* mutant(s) of step (b) in a fermentation medium containing non-volatile thiol precursor(s), under conditions allowing fermentation by the *URE2* mutant(s) to occur, and thereby release of the volatile thiol(s) by the mutant during fermentation,
(d) measuring the volatile thiol(s) released in the fermented medium, by any appropriate means, and
(e) identifying the *URE2* mutant(s) able to release high level volatile thiol, as compared to yeast having a wild-type *URE2* gene.

27. The method of claim 26, wherein the collection of yeasts of step (a) is a collection of yeast strains as defined in anyone of claims 21 to 23.

28. The method of claim 26 or claim 27, wherein the fermentation of step (c) is alcoholic fermentation.

29. The method of anyone of claims 26 to 28, wherein the volatile thiol of step (d) is as defined in claim 25.

30. The method of anyone of claims 20 to 29, wherein the fermented product is as defined in anyone of claims 17 to 19.
